(19) Europäisches Patentamt
European Patent Office
Office européen des brevets

(11) Publication number: 0 311 568 B1

# (12) EUROPEAN PATENT SPECIFICATION

(45) Date of publication of patent specification: 04.09.91 Bulletin 91/36

(51) Int. Cl.[5]: C07C 291/02, C08K 5/32

(21) Application number: 88810673.9

(22) Date of filing: 29.09.88

## (54) Long chain N-alykl-alpha-alkyl nitrones and their use as stabilizers.

(30) Priority: 07.10.87 US 105417

(43) Date of publication of application: 12.04.89 Bulletin 89/15

(45) Publication of the grant of the patent: 04.09.91 Bulletin 91/36

(84) Designated Contracting States: BE DE FR GB IT NL

(56) References cited:
CH-A- 405 268
GB-A- 2 137 619

(73) Proprietor: CIBA-GEIGY AG
Klybeckstrasse 141
CH-4002 Basel (CH)

(72) Inventor: Ravichandran, Ramanathan
111 DeHaven Drive Apt. 125
Yonkers, N.Y. 10703 (US)
Inventor: Pastor, Stephen D.
Neuensteinerstrasse 27
CH-4053 Basel (CH)
Inventor: Seltzer, Raymond
11 Angus Lane
New City, N.Y. 10956 (US)
Inventor: Patel, Ambelal R.
114 Huntley Drive
Ardsley, N.Y. 10502 (US)

EP 0 311 568 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention pertains to novel long chain N-alkyl-alpha-alkyl nitrones, their use as stabilizers for organic materials and the organic materials being stabilized against thermal, oxidative or actinic degradation.

L.P. Nethsinghe et al, Rubber Chem. Tech. 57, 779 (1984) show that N-lower alkyl-alpha-phenyl or substituted phenyl nitrones are effective antifatigue, antioxidant and antiozonant agents for vulcanized rubber.

K.B. Chakraborty et al, J. Appl. Poly. Sci. 30, 3267 (1985) disclose aldonitrones containing a phenolic group which are effective as melt stabilizers for polypropylene.

A.Y. Gerchikov et al, Zh. Prikl. Khim. 1986, 1082 (Chemical Abstracts 106 : 4388n (1987)) describe amide substituted nitrones as antioxidants for industrial esters derived from pentaerythritol.

JP-A-84/11345 describes the melt stabilization of olefin polymers using selected aldonitrones and ketonitrones and US-A-4709107 describes a process for preparing dialkylnitrones.

M. Schulz et al, Plaste Kautsch. 1986, 209 disclose the antioxidant activity of N-phenyl aldonitrones or ketonitrones as antioxidants for polypropylene.

The present invention relates to compounds of the formula I

$$T-\overset{\overset{\displaystyle O^-}{|}}{N^+}=C(G)E \qquad (I)$$

wherein T is a straight or branched chain alkyl of 8 to 18 carbon atoms, G is hydrogen, methyl or ethyl, E is a straight or branched chain alkyl of 5 to 17 carbon atoms, and where the sum of the carbon atoms in G plus E is equal to 7 to 17.

Those compounds of the formula I are preferred, wherein T is n-octyl, n-decyl, dodecyl, tetradecyl, hexadecyl, heptadecyl or octadecyl or is the alkyl mixture found in hydrogenated tallow amine.

Most preferably T is the alkyl mixture found in hydrogenated tallow amine.

Preferably G is hydrogen or methyl. Most preferably G is hydrogen.

Those compounds of the formula I are also preferred, wherein E is n-heptyl, n-nonyl, undecyl, tridecyl, pentadecyl, hexadecyl or heptadecyl or is the alkyl mixture found in hydrogenated tallow amine with one less carbon atom per alkyl group.

Most preferably E is the alkyl mixture found in hydrogenated tallow amine with one less carbon atom per alkyl group.

Those compounds of the formula I are especially preferred, wherein T is the alkyl mixture found in hydrogenated tallow amine, G is hydrogen, and E is the alkyl mixture found in hydrogenated tallow amine with one less carbon atom per alkyl group.

Preferred examples of compounds of the formula I are N-decyl-alpha-nonyl nitrone and N-octadecyl-alpha-heptadecyl nitrone.

A typical di(hydrogenated tallow)amine has the following distribution of alkyl substituents :

$L_1L_2NH$

| $L_1$ | $L_2$ | % |
|---|---|---|
| $C_{16}$ | $C_{14}$ | 1.9 |
| $C_{16}$ | $C_{16}$ | 12.4 |
| $C_{16}$ | $C_{17}$ | 2.8 |
| $C_{16}$ | $C_{18}$ | 36.0 |
| $C_{17}$ | $C_{18}$ | 3.9 |
| $C_{18}$ | $C_{18}$ | 39.0 |
| other | | 4.0 |

It is clear that the di(hydrogenated tallow)amine originating from animal sources may well vary somewhat in the specific distribution of alkyl substituents, but the di(hydrogenated tallow)amine contains major amounts

of N,N-dihexadecylamine, N,N-dioctadecylamine and N-hexadecyl-N-octadecylamine. The individual components of the mixture can be separated by distillation under high vacuum.

However, for the purposes of this invention, there is no need to carry out such separation. Indeed, the di(hydrogenated tallow)amine represents a preferred starting material for preparing the instant nitrones.

The instant nitrones can be prepared by a number of methods. These include (a) reaction of a long chain alkyl aldehyde or ketone with a long chain N-alkylhydroxylamine ; or (b) the oxidation of a long chain N,N-dialkylamine or a long chain N,N-dialkylhydroxylamine with any classic oxidizing agent. Suitable oxidizing agents include aqueous hydrogen peroxide, benzoyl peroxide, tert-butyl hydroperoxide, cumene hydroperoxide and 3-chloroperoxybenzoic acid.

The intermediates used for these synthetic methods are largely items of commerce.

The nitrones of the formula I are excellent process stabilizers for organic materials, in particular synthetic polymers, e.g. polyolefins.

Accordingly, a further embodiment of the present invention is a composition comprising an organic material subject to thermal, oxidative or actinic degradation and at least one compound of the formula I.

In general organic materials which can be stabilized include :

1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optionally can be crosslinked), for example high density polyethylene (HDPE), low density polyethylene (LDPE) and linear low density polyethylene (LLDPE).

2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).

3. Copolymers of monoolefines and diolefines with each other or with other vinyl monomers, such as, for example, ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/ acrylic acid copolymers and their salts (ionomers) and terpolymers of ethylene with propylene and a diene, such as hexadiene, dicyclopentadiene or ethylidene-norbornene ; as well as mixtures of such copolymers and their mixtures with polymers mentioned in 1) above, for example polypropylene/ethylene-propylene-copolymers, LDPE/EVA, LDPE/EAA, LLDPE/EVA and LLDPE/EAA.

3a. Hydrocarbon resins (for example $C_5$-$C_9$) and hydrogenated modifications thereof (for example tackyfiers).

4. Polystyrene, poly-(p-methylstyrene), poly-(α-methylstyrene).

5. Copolymers of styrene or α-methylstyrene with dienes or acrylic derivatives, such as, for example, styrene/butadiene, styrene/ acrylonitrile, styrene/alkyl methacrylate, styrene/maleic anhydride, styrene/butadiene/ethyl acrylate, styrene/acrylonitrile/methyl acrylate ; mixtures of high impact strength from styrene copolymers and another polymer, such as, for example, from a polyacrylate, a diene polymer or an ethylene/propylene/diene terpolymer ; and block copolymers of styrene, such as, for example, styrene/butadiene/ styrene, styrene/ isoprene/styrene, styrene/ethylene/butylene/ styrene or styrene/ ethylene/propylene/styrene.

6. Graft copolymers of styrene or α-methylstyrene such as, for example, styrene on polybutadiene, styrene on polybutadiene-styrene or polybutadiene-acrylonitrile ; styrene and acrylonitrile (or methacrylonitrile) on polybutadiene ; styrene and maleic anhydride or maleimide on polybutadiene ; styrene, acrylonitrile and maleic anhydride or maleimide on polybutadiene ; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS—, MBS—, ASA— or AES-polymers.

7. Halogen-containing polymers, such as polychloroprene, chlorinated chlorinated or sulfochlorinated polyethylene, epichlorohydrin homo- and copolymers, polymers from halogen-containing vinyl compounds,as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/vinyl acetate copolymers.

8. Polymers which are derived from α,β-unsaturated acids and derivatives thereof, such as polyacrylates and polymethacrylates, polyacrylamide and polyacrylonitrile.

9. Copolymers from the monomers mentioned under 8) with each other or with other unsaturated monomers, such as, for instance, acrylonitrile/butadiene, acrylonitrile/alkyl acrylate, acrylonitrile/ alkoxyalkyl acrylate or acrylonitrile/vinyl halogenide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate polyvinyl benzoate, polyvinyl maleate, polyvinyl butyral, polyallyl phthalate or polyallylmelamine ; as well as their copolymers with olefins mentioned in 1) above.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as a comonomer ; polyacetals modified with thermoplastic polyurethanes, acrylates or MBS.
13. Polyphenylene oxides and sulfides, and mixtures of polyphenylene oxides with polystyrene or polyamides.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene diamine and adipic acid ; polyamides prepared from hexamethylenediamine and isophthalic or/and terephthalic acid and optionally an elastomer as modifier, for example poly-2,4,4-trimethylhexamethylene terephthalamide or poly-m-phenylene isophthalamide. Further copolymers of the aforementioned polyamides with polyolefins, olefin copolymers, ionomers or chemically bonded or grafted elastomers ; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly-[2,2-(4-hydroxyphenyl)-propane] terephthalate and polyhydroxybenzoates as well as block-copolyether-esters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or the cellulose ethers, such as methylcellulose ; rosins and their derivatives.
27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylates, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.
29. Aqueous emulsions of natural or synthetic rubber, e.g. natural latex or latices of carboxylated styrene/butadiene copolymers.

Preferably the stabilized organic materials of the present compositions are synthetic polymers, in particular polyolefins.

The polyolefin of the instant compositions is e.g. a homopolymer or copolymer of an alpha-olefin.

The polyolefins useful in the instant compositions are preferably the polymers derived from monoolefins, such as polyethylene, which can be crosslinked, polypropylene, polyisobutylene, polybutene-1, poly-3-methylbutene-1 and poly-4-methylpentene-1. Polyethylene may be for example medium density, high density or linear low density polyethylene.

Mixtures of the homopolymers cited above, for example mixtures of polypropylene and polyethylene, polypropylene and polybutene-1, or polypropylene and polyisobutylene and the like, may also preferably be used.

Copolymers of monoolefins may especially be used in the instant compositions, for example ethylene/propylene copolymers, propylene/butene-1 copolymers, propylene/octene-1 copolymers, ethylene/butene-1 copolymers, ethylene/octene-1 copolymers as well as ethylene/vinyl acetate copolymers.

The instant compositions particularly employ as the polyolefin component polyethylene, polypropylene, polyisobutylene, poly(butene-1), poly(pentene-1), poly(3-methylbutene-1), poly(4-methylpentene-1) and various ethylene and propylene copolymers.

Especially preferred polyolefin substrates are polypropylene, low density polyethylene, medium density polyethylene, high density polyethylene, linear low density polyethylene, poly(butene-1), ethylene/vinyl acetate copolymer, ethylene/propylene copolymer and copolymers of ethylene or of propylene with other alpha-olefins.

The most preferred polyolefin substrate is polypropylene, high density polyethylene, ethylene/propylene copolymer or a copolymer of ethylene or of propylene with another alpha-olefin.

The polyolefins used in the food wrapping industry are of particular interest in these compositions.

The nitrones of this invention stabilize polyolefins especially during high temperature processing with relatively little change in color and melt flow values even though the polymer may undergo a number of extrusions.

In general, the nitrone stabilizers of this invention are employed e.g. from about 0.01 to about 5% by weight of the stabilized composition, although this will vary with the particular substrate and application. An advantageous range is from about 0.025 to about 2%, and especially 0.05 to about 1%.

The instant stabilizers may be readily incorporated into the organic materials by conventional techniques at any convenient stage prior to the manufacture of shaped articles therefrom. For example, the stabilizer may be mixed with the polymer in dry powder form, or a suspension or emulsion of the stabilizer may be mixed with a solution, suspension or emulsion of the polymer.

It is also possible to apply the instant stabilizers by a topical application to the finished articles. This is particularly useful with fiber applications where the instant stabilizers are applied topically to the fibers, for example, by way of a spin finish during the melt spinning process.

The stabilized compositions of the invention may optionally also contain e.g. from about 0.01 to about 5%, preferably from about 0.025 to about 2%, and especially from about 0.1 to about 1% by weight of various conventional additives or stabilizers.

Therefore, the instant invention also pertains to organic materials which, in addition to a nitrone of the formula I, also comprises a stabilizer or mixture of stabilizers selected from the group consisting of the phenolic antioxidants, the hindered amine light stabilizers, the ultraviolet light absorbers, the organic phosphorus compounds, the alkaline metal salts of fatty acids, the thiosynergist and the hydroxylamines.

The phenolic antioxidants useful in such instant compositions embrace a large family of compounds examples of which are given below :

Simple 2,6-dialkylphenols, such as, for example, 2,6-di-tert-butyl-4-methylphenol, 2-tert-butyl-4,6-dimethylphenol, 2,6-di-tert-butyl-4-methoxymethylphenol, 2,6-dioctadecyl-4-methylphenol and 2,6-di-tert-butylphenol.

Derivatives of alkylated hydroquinones, such as, for example, 2,5-di-tert-butyl-hydroquinone, 2,5-di-tert-amyl-hydroquinone, 2,6-di-tert-butyl-hydroquinone, 2,6-di-tert-butyl-4-hydroxyanisole, 3,5-di-tert-butyl-4-hydroxyanisole, tris(3,5-di-tert-butyl-4-hydroxyphenyl)phosphite, 3,5-di-tert-butyl-4-hydroxyphenyl stearate and bis(3,5-di-tert-butyl-4-hydroxyphenyl)adipate.

Hydroxylated thiodiphenyl ethers, such as, for example, 2,2'-thio-bis-(6-tert-butyl-4-methylphenol), 2,2'-thio-bis(4-octylphenol), 4,4'-thio-bis(6-tert-butyl-3-methylphenol), 4,4'-thio-bis(3,6-di-sec-amylphenol), 4,4'-thio-bis(6-tert-butyl-2-methylphenol) and 4,4'-bis(2,6-dimethyl-4-hydroxyphenyl)disulfide.

Alkylidene-bisphenols, such as, for example, 2,2'-methylene-bis(6-tert-butyl-4-methylphenol), 2,2'-methylene-bis(6-tert-butyl-4-ethylphenol), 4,4'-methylene-bis(6-tert-butyl-2-methylphenol), 4,4'-methylene-bis(2,6-di-tert-butylphenol), 2,6-bis(3-tert-butyl-5-methyl-2-hydroxybenzyl)4-methylphenol, 2,2'-methylene-bis[4-methyl-6-(alpha-methylcyclohexyl)-phenol], 1,1-bis(3,5-dimethyl-2-hydroxyphenyl)butane, 1,1-bis(5-tert-butyl-4-hydroxy-2-methylphenyl)butane, 2,2-bis(3,5-di-tert-butyl-4-hydroxyphenyl)propane, 1,1,3-tris(5-tert-butyl-4-hydroxy-2-methylphenyl)-butane,

2,2-bis(5-tert-butyl-4-hydroxy-2-methylphenyl)-4-n-dodecyl-mercaptobutane, 1,1,5,5-tetrakis(5-tert-butyl-4-hydroxy-2-methylphenyl)-pentane and ethylene bis([3,3-bis(3-tert-butyl-4-hydroxyphenyl)-butyrate].

O—, N—and S-benzyl compounds, such as, for example, 3,5,3',5'-tetra-tert-butyl-4,4'-dihydroxydibenzyl ether, octadecyl 4-hydroxy-3,5-dimethylbenzyl-mercaptoacetate, tris(3,5-di-tert-butyl-4-hydroxybenzyl)amine and bis(4-tert-butyl-3-hydroxy-2,6-dimethylbenzyl)dithioterephthalate.

Hydroxybenzylated malonates, such as, for example, dioctadecyl 2,2-bis-(3,5-di-tert-butyl-2-hydroxybenzyl)malonate, dioctadecyl 2-(3-tert-butyl-4-hydroxy-5-methylbenzyl)malonate, bis(dodecylmercapto)ethyl 2,2-bis(3,5-di-tert-butyl-4-hydroxybenzyl)malonate and bis[4-(1,1,3,3-tetramethylbutyl)phenyl] 2,2-bis(3,5-di-tert-butyl-4-hydroxybenzyl)malonate.

Hydroxybenzyl-aromatic compounds, such as, for example, 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene and 2,4,6-tris-(3,5-di-tert-butyl-4-hydroxybenzyl)phenol.

s-Triazine compounds, such as, for example, 2,4-bis(octylmercapto)-6-(3,5-di-tert-butyl-4-hydroxyanilino)-s-triazine, 2-octylmercapto-4,6-bis(3,5-di-tert-butyl-4-hydroxyanilino)-s-triazine, 2-octylmercapto-4,6-bis(3,5-di-tert-butyl-4-hydroxyphenoxy)-s-triazine, 2,4,6-tris-(3,5-di-tert-butyl-4-hydroxyphenoxy)-s-triazine, 2,4,6-tris(3,5-di-tert-butyl-4-hydroxyphenylethyl)-s-triazine, 1,3,5-tris(2,6-dimethyl-3-hydroxy-4-tert-butylbenzyl)isocyanurate and 1,3,5-tris(3,5-di-tert-butyl-4-hydroxybenzyl)isocyanurate.

Amides of β-(3,5-di-tert-butyl-4-hydroxyphenyl)propionic acid, such as, for example, 1,3,5-tris[(3,5-di-tert-butyl-4-hydroxyphenyl)propionyl]-hexahydro-s-triazine and N,N'-bis(3,5-di-tert-butyl-4-hydroxyphenylpropionyl)hexamethylenediamine, N,N'-bis[3-(3,5-di-tert-butyl-4-hydroxyphenyl)propionyl]hydrazine.

Esters of β-(3,5-di-tert-butyl-4-hydroxyphenyl)propionic acid with monohydric or polyhydric alcohols such as, for example, with methanol, ethanol, octadecanol, 1,6-hexanediol, 1,9-nonanediol, ethylene glycol, 1,2-propanediol, diethylene glycol, thiodiethylene glycol, triethylene glycol, neopentylglycol, pentaerythritol, 3-thiaundecanol, 3-thiapentadecanol, trimethylhexanediol, trimethylolethane, trimethylolpropane, tris[hydroxyethyl]isocyanurate and 4-hydroxymethyl-1-phospha-2,6,7-trioxabicyclo[2.2.2]octane.

Esters of β-(5-tert-butyl-4-hydroxy-3-methylphenyl)propionic acid with monohydric or polyhydric alcohols, such as, for example, with methanol ethanol, octadecanol, 1,6-hexanediol, 1,9-nonanediol, ethylene glycol, 1,2-propanediol, diethylene glycol, triethylene glycol, thiodiethylene glycol, neopentylglycol, pentaerythritol, 3-thiaundecanol, 3-thiapentadecanol, trimethylhexanediol, trimethylolethane, trimethylolpropane, tris[hydroxyethyl]isocyanurate and 4-hydroxymethyl-1-phospha-2,6,7-trioxabicyclo[2.2.2]octane.

Esters of 3,5-di-tert-butyl-4-hydroxyphenylacetic acid with monohydric or polyhydric alcohols, such as, for example, with methanol, ethanol, octadecanol, 1,6-hexanediol, 1,9-nonanediol, ethylene glycol, 1,2-propenediol, diethylene glycol, thiodiethylene glycol, neopentylglycol, pentaerythritol, 3-thiaundecanol, 3-thiapentadecanol, trimethylhexanediol, trimethylolethane, trimethylolpropane, tris[hydroxyethyl]isocyanurate and 4-hydroxymethyl-1-phospha-2,6,7-trioxabicyclo[2.2.2]-octane, especially the tetrakis ester of pentaerythritol.

Benzylphosphonates, such as, for example, dimethyl 3,5-di-tert-butyl-4-hydroxybenzylphosphonate, diethyl 3,5-di-tert-butyl-4-hydroxybenzylphosphonate, dioctadecyl 3,5-di-tert-butyl-4-hydroxybenzylphosphonate and dioctadecyl tert-butyl-4-hydroxy-3-methylbenzylphosphonate.

The phenolic antioxidants of particular interest are n-octadecyl 3,5-di-tert-butyl-4-hydroxyhydrocinnamate, neopentanetetrayl tetrakis(3,5-di-tert-butyl-4-hydroxyhydrocinnamate), di-n-octadecyl 3,5-di-tert-butyl-4-hydroxybenzylphosphonate, 1,3,5-tris(3,5-di-tert-butyl-4-hydroxybenzyl)isocyanurate, thiodiethylene bis(3,5-di-tert-butyl-4-hydroxyhydrocinnamate), 1,3,5-trimethyl-2,4,6-tris(3,5-di-tert-butyl-4-hydroxybenzyl)benzene, 3,6-dioxaoctamethylene bis(3-methyl-5-tert-butyl-4-hydroxyhydrocinnamate),2,6-di-tert-butyl-p-cresol, 2,2'-ethylidene-bis-(4,6-di-tert-butylphenol), 1,3,5-tris(2,6-dimethyl-4-tert-butyl-3-hydroxybenzyl)isocyanurate, 1,1,3-tris(2-methyl-4-hydroxy-5-tert-butylphenyl)butane, 1,3,5-tris[2-(3,5-di-tert-butyl-4-hydroxyhydrocinnamoyloxy)ethyl]isocyanurate, 3,5-bis(3,5-di-tert-butyl-4-hydroxybenzyl)mesitol, hexamethylene bis(3,5-di-tert-butyl-4-hydroxyhydrocinnamate), 1-(3,5-di-tert-butyl-4-hydroxyanilino)-3,5-bis(octylthio)-s-triazine, N,N'-hexamethylene-bis(3,5-di-tert-butyl-4-hydroxyhydrocinnamamide), calcium bis(ethyl-3,5-di-tert-butyl-4-hydroxybenzylphosphonate), ethylene bis(3,3-bis(3-tert-butyl-4-hydroxyphenyl)butyrate], octyl 3,5-di-tert-butyl-4-hydroxybenzylmercaptoacetate, bis(3,5-di-tert-butyl-4-hydroxyhydrocinnamoyl)hydrazide, and N,N'-bis[2-(3,5-di-tert-butyl-4-hydroxyhydrocinnamoyloxy)ethyl]oxamide.

A most preferred phenolic antioxidant is neopentanetetrayl tetrakis-(3,5-di-tert-butyl-4-hydroxyhydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-di-tert-butyl-4-hydroxybenzyl)isocyanurate, 2,6-di-tert-butyl-p-cresol or 2,2'-ethylidene-bis-(4,6-di-tert-butylphenol).

When the instant compositions contain a hindered amine light stabilizer, such hindered amines may be for example 4-benzoyl-2,2,6,6-tetramethylpiperidine, 4-stearyloxy-2,2,6,6-tetramethylpiperidine, bis(2,2,6,6-tetramethylpiperidyl)sebacate or 3-n-octyl-7,7,9,9-tetramethyl-1,3,8-triaza-spiro[4.5]decane-2,4-dione.

The hindered amine light stabilizer of particular interest is selected from the group consisting of bis(2,2,6,6-tetramethylpiperidin-4-yl) sebacate, bis(1,2,2,6,6-pentamethylpiperidin-4-yl)sebacate, bis-(1,2,2,6,6-pentamethylpiperidin-4-yl) (3,5-di-tert-butyl-4-hydroxybenzyl)butylmalonate, tris(2,2,6,6-tetramethylpiperidin-4-yl)nitrilotriacetate, 1,2-bis(2,2,6,6-tetramethyl-3-oxopiperazin-4-yl)-ethane, 2,2,4,4-tetramethyl-7-oxa-3,20-diaza-21-oxodispiro[5.1.11.2]heneicosane, polycondensation product of 2,4-dichloro-6-tert-octylamino-s-triazine and 4,4′-hexamethylene-bis(amino-2,2,6,6-tetramethylpiperidine), polycondensation product of 1-(2-hydroxyethyl)-2,2,6,6-tetramethyl-4-hydroxypiperidine and succinic acid, polycondensation product of 4,4′-hexamethylene-bis(amino-2,2,6,6-tetramethylpiperidine) and 1,2-dibromoethane, polycondensation product of 2,4-dichloro-6-morpholino-s-triazine and 4,4′-hexamethylene-bis(amino-2,2,6,6-tetramethylpiperidine), N,N′,N″,N‴-tetrakis[4,6-bis(butyl-(2,2,6,6-tetramethylpiperidin-4-yl)amino)-s-triazin-2-yl]-1,10-diamino-4,7-di azadecane, octamethylene bis(2,2,6,6-tetramethylpiperidin-4-carboxylate) and 4,4′-ethylene-bis-(2,2,6,6-tetramethylpiperazin-3-one).

A most preferred hindered amine light stabilizer in bis(2,2,6,6-tetramethylpiperidin-4-yl)sebacate, the polycondensation product of 1-(2-hydroxyethyl)-2,2,6,6-tetramethyl-4-hydroxypiperidine and succinic acid, the polycondensation product of 2,4-dichloro-6-tert-octylamino-s-triazine and 4,4′-hexamethylene-bis(amino-2,2,6,6-tetramethylpiperidine) or N,N′,N″,N‴-tetrakis[4,6-bis(butyl-(2,2,6,6-tetramethylpiperidin-4-yl)amino)-s-triazin-2-yl]-1,10-diamino-4,7-diazadecane.

When the instant compositions contain an ultraviolet light absorber, such light absorbers may include the 2H-benzotriazoles, the benzophenones, the oxanilides, the alpha-cyanocinnamates, the substituted benzoate esters or the nickel salts of the O-alkyl hindered phenolic benzylphosphonates.

Examples of such ultraviolet light absorbers are seen below :

<u>2-(2′-Hydroxyphenyl)benzotriazoles,</u> e.g., the 5′-methyl-, 3′,5′-di-tert-butyl-, 5′-tert-butyl-, 5′-(1,1,3,3-tetramethylbutyl)-, 5-chloro-3′,5′-di-tert-butyl-, 5-chloro-3′-tert-butyl-5′-methyl-, 3′-sec-butyl-5′-tert-butyl-, 3′-alpha-methyl-benzyl-5′-methyl-, 3′-alpha-methyl-benzyl-5′-methyl-5-chloro-, 4′-hydroxy-, 4′-methoxy, 4′-octyloxy-, 3′,5′-di-tert-amyl-, 3′-methyl-5′-carbomethoxyethyl- and 5-chloro-3′,5′-di-tert-amyl-derivative.

<u>2,4-Bis(2′-hydroxyphenyl)-6-alkyl-s-triazines,</u> e.g., the 6-ethyl-, 6-heptadecyl- or 6-undecyl-derivative.

<u>2-Hydroxybenzophenones,</u> e.g., the 4-hydroxy-, 4-methoxy-, 4-octyloxy-, 4-decyloxy-, 4-dodecyloxy-, 4-benzyloxy-, 4,2′,4′-trihydroxy, 2,2′,4,4′-tetrahydroxy- or 2′-hydroxy-4,4′-dimethoxy-derivative.

<u>1,3-Bis(2′-hydroxybenzoyl)benzenes,</u> e.g., 1,3-bis(2′-hydroxy-4′-hexyloxybenzoyl)benzene, 1,3-bis(2′-hydroxy-4′-octyloxybenzoyl)benzene or 1,3-bis(2′-hydroxy-4′-dodecyloxybenzoyl)benzene.

<u>Esters of optionally substituted benzoic acids,</u> e.g., phenyl salicylate, octylphenyl salicylate, dibenzoylresorcin, bis(4-tert-butylbenzoyl)resorcin, benzoylresorcin, 3,5-di-tert-butyl-4-hydroxybenzoic acid 2,4-di-tert-butylphenyl ester or 2-octadecyl ester or 2-methyl-4,6-di-tert-butyl ester.

<u>Acrylates,</u> e.g., alpha-cyano-β,β-diphenylacrylic acid-ethyl ester or isooctyl ester, alpha-carbomethoxy-cinnamic acid methyl ester, alpha-cyano-β-methyl-p-methoxy-cinnamic acid methyl ester or butyl ester or N-(β-carbomethoxyvinyl)-2-methyl-indoline.

<u>Oxalic acid diamides,</u> e.g., 4,4′-dioctyloxy-oxanilide, 2,2′-dioctyloxy-5,5′-di-tert-butyloxanilide, 2,2′-didodecyloxy-5,5′-di-tert-butyl-oxanilide, 2-ethoxy-2′-ethyl-oxanilide, N,N′-bis(3-dimethylaminopropyl)oxalamide, 2-ethoxy-5-tert-butyl-2′-ethyloxanilide and the mixture thereof with 2-ethoxy-2′-ethyl-5,4′-di-tert-butyl-oxanilide, or mixtures of ortho- and para-methoxy- as well as of o- and p-ethoxy-disubstituted oxanilides.

Preferably the ultraviolet light absorber useful in the instant compositions is 2-(2-hydroxy-5-methylphenyl)-2H-benzotriazole, 2-(2-hydroxy-3,5-di-tert-amylphenyl)-2H-benzotriazole, 2-[2-hydroxy-3,5-bis-(alpha,alpha-dimethylbenzyl)phenyl]-2H-benzotriazole, 2-(2-hydroxy-5-tert-octylphenyl)-2H-benzotriazole, 2-hydroxy-4-octyloxy-benzophenone, nickel bis(O-ethyl 3,5-di-tert-butyl-4-hydroxybenzylphosphonate), 2,4-dihydroxybenzophenone, 2-(2-hydroxy-3-tert-butyl-5-methylphenyl)-2H-benzotriazole, nickel butylamine complex with 2,2′-thiobis(4-tert-butylphenol), 2-ethoxy-2′-ethyloxanilide or 2-ethoxy-2′-ethyl-5,5′-di-tert-butyloxanilide.

When the instant compositions contain an organic phosphorus compound, such compounds may be, for example, triphenyl phosphite, diphenylalkyl phosphites, phenyldialkyl phosphites, tris(nonylphenyl)phosphite, trilauryl phosphite, trioctadecyl phosphite, bis(isodecyloxy)-2,4,8,10-tetraoxa-3,9-diphospha[5.5]undecane and tris(4-hydroxy-3,5-di-tert-butylphenyl)phosphite or similar phosphonites.

The organic phosphorus compound of particular interest is selected from the group consisting of tris(2,4-di-tert-butylphenyl)phosphite, 3,9-bis-(2,4-di-tert-butylphenoxy)-2,4,8,10-tetraoxa-3,9-diphospha[5.5]undecane, tris(p-nonylphenyl)phosphite, 3,9-distearyloxy-2,4,8,10-tetraoxa-3,9-diphospha[5.5]undecane, dilauryl phosphite, 3,9-bis[2,6-di-tert-butyl-4-(2-(n-octadecyloxycarbonyl)ethyl)phenoxy]-2,4,8,10-tetraoxa-3,9-diphospha[5.5]undecane and tetrakis(2,4-di-tert-butylphenyl)-4,4′-bis[diphenylene phosphonite]. Tris(2,4-di-tert-butylphenyl)phosphite is especially preferred.

The alkaline metal salts of a fatty acid useful in the instant compositions are the alkali metal, alkaline earth metal, zinc, cadmium or aluminum salts of the higher fatty acids, for example calcium stearate, zinc stearate, magnesium behenate, sodium ricinoleate or potassium palmitate. Calcium stearate and zinc stearate are particularly preferred.

When the instant compositions contain a thiosynergist, such thiosynergists may be for example dilauryl thiodipropionate, distearyl thiodipropionate or neopentanetetrayl tetrakis(3-dodecylthiopropionate). Distearyl thiodipropionate or dilauryl thiodipropionate is particularly preferred.

When the instant compositions contain a hydroxylamine, such hydroxylamine may be for example an N,N-diaralkylhydroxylamine, an N,N-dicycloalkylhydroxylamine or an N,N-dialkylhydroxylamine. Preferred examples of such hydroxylamines include N,N-dibenzylhydroxylamine, substituted N,N-dibenzylhydroxylamines, N,N-dicyclohexylhydroxylamine and the N,N-dialkylhydroxylamines where alkyl is of 8 to 18 carbon atoms.

The following may be mentioned as examples of further additives that can be used in the instant compositions.

Metal deactivators, e.g., oxanilide, isophthalic acid dihydrazide, sebacic acid bis[phenylhydrazide], bis-benzylidene-oxalic acid hydrazide, N,N'-diacetal adipic acid dihydrazide, N,N'-bis(salicyloyl)oxalic acid dihydrazide, N,N'-bis(salicyloyl)hydrazine, N,N'-bis(3,5-di-tert-butyl-4-hydroxyphenylpropionyl)hydrazine, N-salicyloyl-N'-salicylalhydrazine, 3-salicyloyl-amino-1,2,4-triazole or N,N'-bis(salicyloyl)thiopropionic acid dihydrazide.

Nucleation agents, e.g., 4-tert-butylbenzoic acid, adipic acid, diphenylacetic acid or substituted sorbitols such as 1,3,2,4-dibenzylidenesorbitol.

Other additives that can be incorporated in the stabilized compositions are antiblocking agents, clarifiers, antiozonants, lubricants such as e.g. stearyl alcohol, fillers, carbon black, asbestos, kaolin, talc, glass fibers, pigments, optical brighteners, flameproofing agents and antistatic agents.

A particularly preferred embodiment of the instant invention is an organic material which, in addition to a nitrone of the formula I, also comprises an alkaline metal salt of a fatty acid and/or a phenolic antioxidant.

The Alkaline metal salt of a fatty acid is preferably employed from 0.01 to 2%, in particular 0.05 to 1%, and especially 0.1 to 0.5% by weight of the stabilized composition.

Another particularly preferred embodiment of the instant invention is an organic material which, in addition to a nitrone of the formula I, also comprises a N,N-dialkylhydroxylamine where alkyl is of 8 to 18 carbon atoms.

Example 1 : Preparation of N-decyl-alpha-nonyl nitrone

To a solution of 29.8 g of didecylamine in 120 ml of n-propanol is added sequentially 1.6 g of sodium tungstate dihydrate and 14.3 g of a 50% aqueous hydrogen peroxide solution at 5-10°C. The reaction mixture is allowed to warm up to 40°C and is maintained at that temperature for 20 hours. An additional 7.2 g of 50% aqueous hydrogen peroxide solution is added to the solution at 40°C. After further stirring for another day, the reaction mixture is concentrated under reduced pressure. The residue is partitioned between chloroform and water. The combined organic extracts are washed with water and then dried over anhydrous sodium sulfate. Removal of the chloroform solvent leaves a crude product which is purified by recrystallization from heptane to afford the above-named compound as a white solid melting at 63-65°C in a yield of 9.8 g.

Analysis:

Calcd. for $C_{20}H_{41}NO$: C, 77.1; H, 13.3; N, 4.5.

Found: C, 77.1; H, 13.7; N, 4.5.

Example 2 : Preparation of N,N-di(hydrogenated tallow)hydroxylamine

Into a solution of 100 g (0.18 mol) of di(hydrogenated tallow)amine (494 eq. wt., 90% secondary amine) in 400 ml of n-butanol at 55°C is added 8.6 ml (0.22 mol) of 70% aqueous hydrogen peroxide solution. The reaction is complete when all the hydrogen peroxide is consumed as determined by titration of an aliquot of the reaction mixture with potassium iodide/sulfuric acid/sodium thiosulfate.

The above-named product is isolated from the reaction mixture by filtration. The filter cake is washed with two 50 ml portions of n-butanol at 55°C ; then dried to give the desired product in a yield of 63 g (68%) as a white solid melting at 93-96°C.

Example 3 : Preparation of nitrone derived from di(hydrogenated tallow)hydroxylamine

To a solution of 3.5 g of di(hydrogenated tallow)hydroxylamine, prepared as in Example 2, in 35 ml of toluene is added 2.8 ml of a 3.65 M tert-butyl hydroperoxide solution in toluene. The resulting solution is heated under reflux for 30 minutes. The reaction mixture is concentrated under reduced pressure. The residue is first purified by recrystallization from isopropanol and then by a second recrystallization from toluene. The above titled nitrone is isolated as a white solid melting at 83-85°C in a yield of 1.5 g.

Analysis:

Calcd. for $C_{36}H_{73}NO$: C, 80.7; H, 13.7; N, 2.6.

Found: C, 81.1; H, 14.2; N, 2.7.

Example 4 : Preparation of nitrone derived from di(hydrogenated tallow)amine

The above named compound is also prepared by the procedure described in Example 1 by the direct oxidation of the di(hydrogenated tallow)amine using 50% aqueous hydrogen peroxide and sodium tungstate.

Example 5 : Preparation of N-octadecyl-alpha-heptadecyl nitrone

To a stirred suspension of 4.0 g (7.4 mmol) of N,N-dioctadecylhydroxylamine and 0.13 g (0.4 mmol) of sodium tungstate dihydrate in 35 ml of isopropyl alcohol is added 0.60 g of 50% aqueous solution of hydrogen peroxide. The reaction mixture is heated for 30 minutes during which time it becomes homogeneous. A TLC analysis conducted on the reaction mixture (9 :1, chloroform :ethyl acetate eluent) indicates that a small amount of starting material is still present.

To the reaction mixture at reflux temperature is then added an additional 0.20 g of 50% aqueous hydrogen peroxide and heating is continued for another 30 minutes. The reaction mixture is then filtered while hot to remove the sodium tungstate catalyst.

The filtrate is cooled to 0°C and is filtered to give 3.58 g (91% yield) of a white solid melting at 93-96.5°C.

Analysis:

Calcd. for $C_{36}H_{73}NO$: C, 80.7; H, 13.7; N, 2.61.

Found: C, 80.3; H, 13.9; N, 2.6.

Example 6 : Process stabilization of polypropylene at 260°C

The base formulation comprises 100 parts of unstabilized polypropylene (®Profax 6501, Himont) with 0.10 parts of calcium stearate. The test stabilizer is solvent blended onto the propylene from a solution in methylene chloride. After removal of the solvent by evaporation under reduced pressure, the stabilized resin formulation is extruded at 100 rpm from a 2.54 cm diameter extruder at 260°C.

After each of the first, third and fifth extrusions, resin pellets are compression molded into 3.2 mm thick plaques at 193°C and specimen yellowness index (YI) is determined according to ASTM D-1925. Low YI values indicate less yellowing. Additionally, the melt flow rate (in g/10 min) according to ASTM D-1238 is measured on the pellets after the first and fifth extrusions. The closer the melt flow rate after the fifth extrusion is to the melt flow rate after the first extrusion the superior the process stabilization of the polypropylene is.

| Stabilizer | Yellowness Index Color After Extrusion | | | Melt Flow Rate After Extrusion | |
|---|---|---|---|---|---|
| | 1 | 3 | 5 | 1 | 5 |
| Base Formulation | 2.2 | 2.4 | 2.5 | 7.0 | 14.6 |
| 0.1 % of Nitrone of Example 1 | 1.8 | 3.3 | 4.5 | 3.4 | 6.5 |
| 0.1 % of Nitrone of Example 3 | 2.1 | 3.1 | 3.2 | 3.6 | 6.2 |

Example 7 : Process stabilization of polypropylene at 260°C

When, following the procedure of Example 6, an equivalent amount of a mixture of the N,N-di(hydrogenated tallow)hydroxylamine and N-octadecyl-alpha-heptadecyl nitrone is used as stabilizer in the polypropylene containing 0.1% of calcium stearate, excellent protection against discoloration and against polymer degradation during processing is obtained.

## Claims

1. A compound of the formula I

$$T-\overset{\overset{O^-}{|}}{N^+}=C(G)E \qquad (I)$$

wherein T is a straight or branched chain alkyl of 8 to 18 carbon atoms, G is hydrogen, methyl or ethyl, E is a straight or branched chain alkyl of 5 to 17 carbon atoms, and where the sum of the carbon atoms in G plus E is equal to 7 to 17.

2. A compound according to claim 1 wherein T is n-octyl, n-decyl, dodecyl, tetradecyl, hexadecyl, heptadecyl or octadecyl or is the alkyl mixture found in hydrogenated tallow amine.

3. A compound according to claim 2 wherein T is the alkyl mixture found in hydrogenated tallow amine.

4. A compound according to claim 1 wherein G is hydrogen or methyl.

5. A compound according to claim 4 wherein G is hydrogen.

6. A compound according to claim 1 wherein E is n-heptyl, n-nonyl, undecyl, tridecyl, pentadecyl, hexadecyl or heptadecyl or is the alkyl mixture found in hydrogenated tallow amine with one less carbon atom per alkyl group.

7. A compound according to claim 6 wherein E is the alkyl mixture found in hydrogenated tallow amine with one less carbon atom per alkyl group.

8. The compound according to claim 1 which is N-decyl-alpha-nonyl nitrone.

9. A compound according to claim 1 wherein T is the alkyl mixture found in hydrogenated tallow amine, G is hydrogen, and E is the alkyl mixture found in hydrogenated tallow amine with one less carbon atom per alkyl group.

10. The compound according to claim 1 which is N-octadecyl-alpha-heptadecyl nitrone.

11. A composition comprising an organic material subject to thermal, oxidative or actinic degradation and at least one compound according to claim 1.

12. A composition according to claim 11 wherein the organic material is a synthetic polymer.

13. A composition according to claim 11 wherein the organic material is a polyolefin.

14. A composition according to claim 13 wherein the polyolefin is selected from the group consisting of polypropylene, low density polyethylene, medium density polyethylene, high density polyethylene, linear low density polyethylene, poly(butene-1), ethylene/vinyl acetate copolymer, ethylene/propylene copolymer and copolymers of ethylene or of propylene with other alpha-olefins.

15. A composition according to claim 11 which additionally contains a stabilizer or mixture of stabilizers selected from the group consisting of the phenolic antioxidants, the hindered amine light stabilizers, the ultraviolet light absorbers, the organic phosphorus compounds, the alkaline metal salts of fatty acids, the thiosynergists and the hydroxylamines.

16. A composition according to claim 11 which additionally contains an alkaline metal salt of a fatty acid and/or a phenolic antioxidant.

17. A composition according to claim 11 which additionally contains a N,N-dialkylhydroxylamine where alkyl is of 8 to 18 carbon atoms.

18. Use of a compound according to claim 1 for stabilizing an organic material against thermal, oxidative or actinic degradation.

## Patentansprüche

1. Eine Verbindung der Formel I,

$$T - \overset{O^{-}}{\overset{|}{N^{+}}} = C(G)E \qquad (I)$$

worin T geradkettiges oder verzweigtes Alkyl mit 8 bis 18 Kohlenstoffatomen bedeutet, G Wasserstoff, Methyl oder Ethyl ist, E geradkettiges oder verzweigtes Alkyl mit 5 bis 17 Kohlenstoffatomen bedeutet und die Summe der Kohlenstoffatome in G plus E gleich 7 bis 17 beträgt.

2. Eine Verbindung gemäss Anspruch 1, worin T n-Octyl, n-Decyl, Dodecyl, Tetradecyl, Hexadecyl, Heptadecyl oder Octadecyl oder die Alkylmischung ist, die in hydriertem Talgamin gefunden wird.

3. Eine Verbindung gemäss Anspruch 2, worin T die Alkylmischung ist, die in hydriertem Talgamin gefunden wird.

4. Eine Verbindung gemäss Anspruch 1, worin G Wasserstoff oder Methyl bedeutet.

5. Eine Verbindung gemäss Anspruch 4, worin G Wasserstoff ist.

6. Eine Verbindung gemäss Anspruch 1, worin E n-Heptyl, n-Nonyl, Undecyl, Tridecyl, Pentadecyl, Hexadecyl oder Heptadecyl bedeutet oder die Alkylmischung ist, die in hydriertem Talgamin mit einem Kohlenstoffatom weniger pro Alkylgruppe gefunden wird.

7. Eine Verbindung gemäss Anspruch 6, worin E die Alkylmischung bedeutet, die in hydriertem Talgamin mit einem Kohlenstoffatom weniger pro Alkylgruppe gefunden wird.

8. Die Verbindung gemäss Anspruch 1, die N-Decyl-alpha-nonylnitron ist.

9. Eine Verbindung gemäss Anspruch 1, worin T die Alkylmischung ist, die in hydriertem Talgamin gefunden wird, G Wasserstoff bedeutet und E die Alkylmischung ist, die in hydriertem Talgamin mit einem Kohlenstoffatom weniger pro Alkylgruppe gefunden wird.

10. Die Verbindung gemäss Anspruch 1, die N-Octadecyl-alpha-heptadecylnitron ist.

11. Eine Zusammensetzung enthaltend ein gegen thermischen, oxidativen oder aktinischen Abbau empfindliches organisches Material und mindestens eine Verbindung gemäss Anspruch 1.

12. Eine Zusammensetzung gemäss Anspruch 11, worin das organische Material ein synthetisches Polymer ist.

13. Eine Zusammensetzung gemäss Anspruch 11, worin das organische Material ein Polyolefin ist.

14. Eine Zusammensetzung gemäss Anspruch 13, worin das Polyolefin ausgewählt wird aus einer Gruppe bestehend aus Polypropylen, Polyethylen niederer Dichte, Polyethylen mittlerer Dichte, Polyethylen hoher Dichte, lineares Polyethylen niederer Dichte, Poly(buten-1), Ethylen-Vinylacetat-Copolymer, Ethylen-Propylen-Copolymer und Copolymeren von Ethylen oder Propylen mit anderen alpha-Olefinen.

15. Eine Zusammensetzung gemäss Anspruch 11, welche zusätzlich einen Stabilisator oder eine Mischung von Stabilisatoren ausgewählt aus der Gruppe bestehend aus den phenolischen Antioxidantien, den gehinderten Amin-Lichtstabilisatoren, den Ultraviolettlicht-Absorbern, den organischen Phosphorverbindungen, den basischen Metallsalzen der Fettsäuren, den Thiosynergisten und den Hydroxylaminen enthält.

16. Eine Zusammensetzung gemäss Anspruch 11, welche zusätzlich ein basisches Metallsalz einer Fettsäure und/oder ein phenolisches Antioxidans enthält.

17. Eine Zusammensetzung gemäss Anspruch 11, welche zusätzlich ein N,N-Dialkylhydroxylamin enthält, worin Alkyl 8 bis 18 Kohlenstoffatome besitzt.

18. Verwendung einer Verbindung gemäss Anspruch 1 zum Stabilisieren von organischem Material gegen

thermischen, oxidativen oder aktinischen Abbau.

## Revendications

1. Composé de formule I

$$T-\overset{\overset{O^-}{|}}{N^+}=C(G)E \qquad (I)$$

où T est un groupe alkyle à chaîne droite ou ramifiée de 8 à 18 atomes de carbone, G est l'hydrogène, un groupe méthyle ou éthyle, E est un groupe alkyle à chaîne droite ou ramifiée de 5 à 17 atomes de carbone, et le nombre total d'atomes de carbone dans G et E est de 7 à 17.

2. Composé selon la revendication 1, dans lequel T est un groupe *n*-octyle, *n*-décyle, dodécyle, tétradécyle, hexadécyle, heptadécyle ou octadécyle, ou bien est le mélange de groupes alkyle existant dans une amine de suif hydrogéné.

3. Composé selon la revendication 2, dans lequel T est le mélange de groupes alkyle existant dans une amine de suif hydrogéné.

4. Composé selon la revendication 1, dans lequel G est l'hydrogène ou le groupe méthyle.

5. Composé selon la revendication 4, dans lequel G est l'hydrogène.

6. Composé selon la revendication 1, dans lequel E est le groupe *n*-heptyle, *n*-nonyle, undécyle, tridécyle, pentadécyle, hexadécyle ou heptadécyle, ou bien est le mélange de groupes alkyle existant dans une amine de suif hydrogéné avec un atome de carbone de moins par groupe alkyle.

7. Composé selon la revendication 6, dans lequel E est le mélange de groupes alkyle existant dans une amine de suif hydrogéné avec un atome de carbone de moins par groupe alkyle.

8. Composé selon la revendication 1, qui est la N-décyl-α-nonylnitrone.

9. Composé selon la revendication 1, dans lequel T est le mélange de groupes alkyle existant dans une amine de suif hydrogéné, G est l'hydrogène et E est le mélange de groupes alkyle existant dans une amine de suif hydrogéné avec un atome de carbone de moins par groupe alkyle.

10. Composé selon la revendication 1, qui est la N-octadécyl-α-heptadécylnitrone.

11. Composition comprenant une matière organique sensible à une dégradation par la chaleur, l'oxydation ou un rayonnement actinique et au moins un composé selon la revendication 1.

12. Composition selon la revendication 11, dans laquelle la matière organique est un polymère synthétique.

13. Composition selon la revendication 11, dans laquelle la matière organique est une polyoléfine.

14. Composition selon la revendication 13, dans laquelle la polyoléfine est choisie dans le groupe formé par le polypropylène, le polyéthylène basse densité, le polyéthylène moyenne densité, le polyéthylène haute densité, le polyéthylène basse densité linéaire, le poly(butène-1), un copolymère éthylène/acétate de vinyle, un copolymère éthylène/propylène et les copolymères d'éthylène ou de propylène avec d'autres α-oléfines.

15. Composition selon la revendication 11, qui contient de plus un stabilisant ou un mélange de stabilisants choisis dans le groupe formé par les antioxydants phénoliques, les stabilisants à la lumière du type amine à empêchement stérique, les absorbants de lumière ultraviolette, les composés organiques du phosphore, les sels de métaux alcalins d'acides gras, les agents de synergie sulfurés et les hydroxylamines.

16. Composition selon la revendication 11, qui contient de plus un sel de métal alcalin d'un acide gras et/ou un antioxydant phénolique.

17. Composition selon la revendication 11, qui contient de plus une N,N-dialkylhydroxylamine dont chaque groupe alkyle compte 8 à 18 atomes de carbone.

18. Utilisation d'un composé selon la revendication 1 pour stabiliser une matière organique contre une dégradation par la chaleur, l'oxydation ou un rayonnement actinique.